# EUROPEAN PATENT APPLICATION

(11) **EP 1 815 870 A1**
(43) Date of publication of application: **08.08.2007**
(21) Application number: 06101156.5
(22) Date of filing: 01.02.2006
(51) Int. Cl.: A61K 49/00, A61K 49/08, A61K 51/04

(54) **Cyanine dye compounds linked to metal chelator for bi-modal diagnostic imaging**

(71) Applicant: DKFZ Deutsches Krebsforschungszentrum, 69117 Heidelberg (DE); Ruprecht-Karls-Universität Heidelberg, 69117 Heidelberg (DE)
(72) Inventor: Kiessling, Fabian, 69120 Heidelberg (DE); Beijer, Barbro, 69226 Nussloch (DE); Schulz, Ralf, 68159 Mannheim (DE); Semmler, Wolfhard, 69118 Heidelberg (DE); Mier, Walter, 64625 Bensheim (DE); Wängler, Björn, 65259 Wilhelmsfeld (DE)
(74) Representative: Huhn, Michael

(57) **Abstract**

The present invention relates to compounds of formula I,

Ch-L-D (I),

wherein D is a cyanine dye, L is a covalent bond or a linker and Ch is a metal chelating moiety or a pharmaceutically acceptable salt thereof, which can be used for the preparation of a contrast agent for the at least bi-modal diagnostic imaging of a biological system as well as a contrast agent prepared from these compounds, their use in multi-modal diagnostic imaging and imaging methods.

## Description

The present invention relates to compounds and their use for the preparation of contrast agents, said contrast agents as well as their use for the at least bi-modal diagnostic imaging and in-vivo diagnostic methods.

Powerful diagnostic imaging techniques using various contrast agents have been developed in the recent years. One type of contrast agent includes a chelating molecule which is capable to coordinate an active metal for the desired imaging application. Such an application may be single-photon-emission computer tomography (SPECT) where a suitable metal is ¹¹¹In^{III}. Another method is called positron emission tomography (PET) where normally ⁶⁸Ga^{III} is used as an active metal. Finally, magnetic resonance imaging (MRI) has to be named as a useful method where Gd^{III} is coordinately bound to a chelator in a contrast agent. MRI offers a high spacial resolution and good contrast results for soft tissue. However, MRI is relatively cost-intensive. Another important radio imaging technique is scintigraphy.

A more inexpensive way is given by using optical imaging techniques like fluorescence reflectance imaging (FRI) or fluorescence mediated tomography (FMT). A disadvantage of these techniques is that no anatomical information can be obtained. On the other side, important functional information are available by optical methods. Here, contrast agents emitting near-infrared (NIR) light are useful tools for the imaging of deeper tissues.

To combine the various methods, dual probes have been developed for optical and MR imaging.

One approach for dual contrast agents makes use of nanoparticles where a chelating moiety and an optical active moiety are attached to a peptidic polymer backbone (L. Josephson et al., Bioconjugate Chem. 13 (2002), 554-560).

M. M. Hüber et al., Bioconjugate Chem. 9 (1998), 242-249, describe similar contrast agents where the active molecule is a poly-D-lysine or a dextran polymer having diethylenetetraminepentaacetic acid (DTPA) as complexing moiety and tetramethylrhodamine as dye for the contrast agent.

S. Quici et al., Inorg. Chem. 41 (2002), 2777-2784, use 1,4,7,10-tetraazacyclododecane-1,4,7-triacetic acid (DO3A) which was functionalized with 1,10-phenanthroline as luminescent chromophore.

Further contrast agents of this type are disclosed in US-A 2003/129579.

Contrast agents for monomodal near-infrared radiation imaging are disclosed in US-A 2001/0055567. Here, a cyanine dye is used as chromophore. This molecule may be functionalized with a hydrophilic group, like a polycarboxylic moiety to improve water-solubility. Although these structures may be used as complexing centers US-A 2001/055567 does not describe the use of such compounds for bi-modal imaging.

Although many contrast agents are described in the state of the art which are capable to be used in multi-modal diagnostic imaging techniques, there is a need for improved contrast agents and especially for compounds that can be used for the preparation of contrast agents for said imaging.

Thus, an object of the present invention is to provide compounds that can be used for the preparation of contrast agents for at least bi-modal diagnostic imaging.

This object is solved by the use of a compound of formula I

Ch-L-D (I),

wherein D is a cyanine dye, L is a covalent bond or a linker and Ch is a metal chelating moiety or a physiologically acceptable salt thereof, for the preparation of a contrast agent for the at least bi-modal diagnostic imaging of a biological system, said at least bi-modal imaging comprising at least an optical and at least a non-optical imaging technique.

Surprisingly, it was found that compounds having a metal chelating moiety and a cyanine dye are useful precursor compounds for the preparation of a contrast agent for the at least bi-modal diagnostic imaging.

A cyanine dye according to the present invention is formed by two nitrogen heterocycles where the nitrogen atoms are connected with a conjugated polymethine chain to form a π-electron system where the nitrogen atoms are used as donator and acceptor centers as well as their oxygen analogues.

Suitable cyanine dyes are disclosed by W. Pham et al., Bioconjugate Chem. 16 (2005), 735-740, A. Becker et al., Nature Biotechnology 19 (2001), 327-331, S. A. Hilderbrand et al., Bioconjugate Chem. 16 (2005), 1275-1281, Y. Lin et al., Bioconjugate Chem. 13 (2002), 605-610, O. Mader et al., Bioconjugate Chem. 15 (2004), 70-78, J. V. Frangioni, Current Opinion in Chemical Biology (2003), 626-634 as well as by Dyomics GmbH, Jena (DE) (Catalogue - Fluorescent Dyes for Bioanalytical and Hightech Applications; 4^{th} Edition - autumn 2005, especially pp. 22-27).

The cyanine dye is directly or indirectly linked to a metal chelating moiety. Suitable chelating moieties are derived from DTPA, DOTA or TETA.

Chelating moieties which can be combined with the cyanine dye to form compounds of the present invention for the preparation of contrast agents are described by M. Li et al., J. Am. Chem. Soc. 117 (1995), 8132-8138, K. R. Raymond et al., Bioconjugate Chem., 16 (2005), 3-8, and S. Aime et al., Chemical Society Reviews, 27 (1998), 19-29.

The compounds of the present invention are useful for the preparation of a contrast agent for the at least bi-modal diagnostic imaging of a biological system, wherein said at least bi-modal imaging comprises at least an optical and at least a non-optical imaging technique.

In a preferred embodiment of the present invention, the at least one optical imaging technique is near-infrared fluorescence.

In a further preferred embodiment, the at least one non-optical imaging technique is selected from the group consisting of nuclear magnetic resonance imaging (NMRI), positron emission tomography (PET), and single photon emission computer tomography (SPECT).

The biological system may be any system capable of being imaged by the afore mentioned diagnostic imaging techniques.

Preferred biological systems are the normal and pathologically altered living body of mammalians and humans or parts thereof, especially tissue, organs, cells and the like.

Since the cyanine dye includes at least one heteroatom with a positive charge, the compounds of the present invention may possess additionally a negatively charged functional group resulting in an inner salt. Otherwise, the compounds of the present invention are provided by an additional counter ion resulting in a pharmaceutical acceptable salt.

"Pharmaceutically acceptable" means that the resulting salt is suitable for the diagnostic imaging and especially do not negatively affect the biological system. Suitable counter ions to be used are known in the art. Suitable counter ions would be halogens, especially Cl⁻, Br⁻ and the sulphate ion (SO₄²⁻).

Preferred compounds of the present invention for the preparation of a contrast agent are those compounds represented by formula II 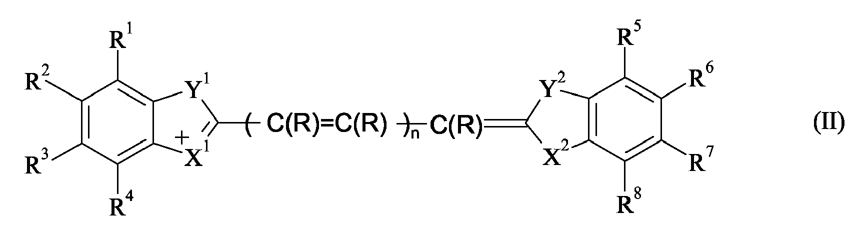 wherein
X¹ is selected from the group consisting of O, N(R¹³), C(R¹⁴)-C(R¹⁵)=O, and C(R¹⁴)-C(R¹⁵)=N(R¹³);
X² is selected from the group consisting of O, and N(R¹³);
Y¹, Y² are independently selected from the group consisting of a covalent single bond, -C(R⁹R¹⁰)-, O, S, -C(R⁹R¹⁰)-C(R¹¹R¹²)-, and -C(R⁹)=C(R¹¹)-;
n is 0, 1, 2, 3, 4 or 5, preferably 1, 2, or 3;
each R is independently selected from the group consisting of hydrogen, halogen, OH, NH₂, C(O)OH, C(O)NH₂, SO₃H, NO₂, CN, Ch-L-, and C₁₋₆ alkyl, wherein C₁₋₆ alkyl is optionally substituted with one or more halogen, OH, NH₂, C(O)OH, C(O)NH₂, SO₃H, NO₂, CN;
optionally two R groups in 1,3-cis position jointly form together with the carbons to which they are attached to a cycle selected from the group consisting of cyclopentene, cyclopentadiene, cyclohexene, and cyclohexadiene, wherein the cycle is optionally substituted with one or more substituents, which are the same or different, selected from the group consisting of halogen, OH, NH₂, C(O)OH, C(O)NH₂, SO₃H, NO₂, CN, Ch-L-, and C₁₋₆ alkyl, wherein C₁₋₆ alkyl is optionally substituted with one or more halogen, OH, NH₂, C(O)OH, C(O)NH₂, SO₃H, NO₂, CN;
R¹, R², R³, R⁴, R⁵, R⁶, R⁷, R⁸, R⁹, R¹⁰, R¹¹, R¹², R¹⁴, R¹⁵ are independently selected from the group consisting of hydrogen, halogen, OH, NH₂, C(O)OH, C(O)NH₂, SO₃H, NO₂, CN, Ch-L-, and C₁₋₆ alkyl, wherein C₁₋₆ alkyl is optionally substituted with one or more halogen, OH, NH₂, C(O)OH, C(O)NH₂, SO₃H, NO₂, CN;
optionally one or more pairs selected from the group consisting of R¹/R², R²/R³, R³/R⁴, R⁵/R⁶, R⁶/R⁷, and R⁷/R⁸ jointly form together with the carbon atoms to which they are attached to a cycle selected from the group consisting of phenyl, naphthyl, heterocycle and heterobicycle;
each R¹³ is independently selected from the group consisting of hydrogen, C(O)OH, C(O)NH₂, Ch-L-, and C₁₋₆ alkyl, wherein C₁₋₆ alkyl is optionally substituted with one or more halogen, OH, NH₂, C(O)OH, C(O)NH₂, SO₃H, NO₂, CN;
provided that one of the groups R, R¹ to R¹⁵ is -L-Ch, wherein L and Ch have the meaning as above.

According to the present invention, C₁₋₆ alkyl means an alkyl group having 1 to 6 carbon atoms. Examples of C₁₋₆ alkyl are methyl, ethyl, n-propyl, i-propyl, n-butyl, sec-butyl, i-butyl, tert.-butyl, n-pentyl, n-hexyl.

Halogen means fluoro, chloro, bromo, jodo, preferably fluoro.

"Heterocyclyl" or "heterocycle" means a cyclopentane, cyclohexane or cycloheptane ring that may contain up to the maximum number of double bonds (aromatic or non-aromatic ring which is fully, partially or un-saturated) wherein at least one carbon atom up to 4 carbon atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for a heterocycle are furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, azepine or homopiperazine. "Heterocycle" means also azetidine.

"Heterobicyclyl" or "heterobicycle" means a heterocycle which is condensed with phenyl, C₃₋₇ cycloalkyl or an additional heterocycle to form a bicyclic ring system. "Condensed" to form a bicyclic ring means that two rings are attached to each other by sharing two ring atoms. Examples for a heterobicycle are indole, indoline, benzofuran, benzothiophene, benzoxazole, benzisoxazole, benzothiazole, benzisothiazole, benzimidazole, benzimidazoline, quinoline, quinazoline, dihydroquinazoline, quinoline, dihydroquinoline, tetrahydroquinoline, decahydroquinoline, isoquinoline, decahydroisoquinoline, tetrahydroisoquinoline, dihydroisoquinoline, benzazepine, purine or pteridine.

The chelating moiety and the dye in the compounds of the present invention are directly attached to each other, i.e. L is a covalent bond, or the groups are spaced apart by a C₁₋₆ alkyl group, which is optionally interrupted by one or more atoms or functional groups selected from the group consisting of S, O, NH, C(O)O, and C(O)NH. L is optionally substituted with one or more substituents independently selected from the group consisting of halogen, OH, NH₂, C(O)H, C(O)NH₂, SO₃H, NO₂, and CN.

In a preferred embodiment, the group CH-L is selected from the group consisting of 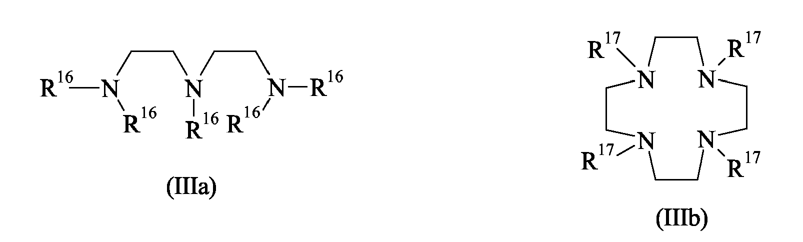 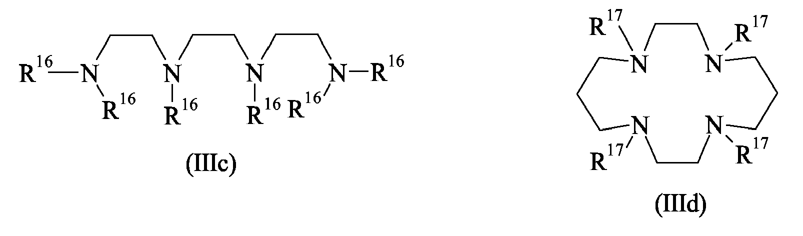 wherein
each R¹⁶ is independently selected from the group consisting of hydrogen, CH₂C(O)OH, CH₂C(O)NH₂, CH₂C(O)NHCH₃, CH₂C(O)NHCH₂Ph, CH₂PO₃H, CH₂P(CH₂Ph)O₂H, CH₂SO₃H, CH₂CH₂OH, C(O)R¹⁸, C(O)CH₂NHC(O)R¹⁸, and deprotonated derivatives thereof;
each R¹⁷ is independently selected from the group consisting of hydrogen, CH₂C(O)OH, CH(CH₂OCH₂Ph)C(O)OH, CH₂C(O)NH₂, CH₂C(O)NHCH₃, CH₂C(O)NHCH₂Ph, CH₂PO₃H, CH₂P(CH₂Ph)O₂H, CH₂SO₃H, CH₂CH₂OH, CH₂CH(OH)CH₃ and deprotonated forms thereof;
R¹⁸ is selected from the group consisting of 3-hydroxy-pyridin-2-on-4-yl, 5-hydroxy-pyrimidin-4-on-6-yl, 1-oxo-pyridin-2-on-6-yl, 1-hydroxy-phen-2-yl, 1,2-dihydroxy-phen-3-yl, wherein R¹⁸ is optionally substituted with one or more further substituents independently selected from the group consisting of CH₃, CH₂CH₂OH, CH₂CH₂OCH₃, C(O)NH₂, and C(O)NHCH₃,
provided that one hydrogen of the compound according to one of the formulae IIIa to IIId is replaced by L and L has the meaning as indicated above.

Preferably, Ch-L is selected from the formulae IIIb and IIId.

Further preferred compounds of the present invention are represented by formulae IIa, IIb and IIc. 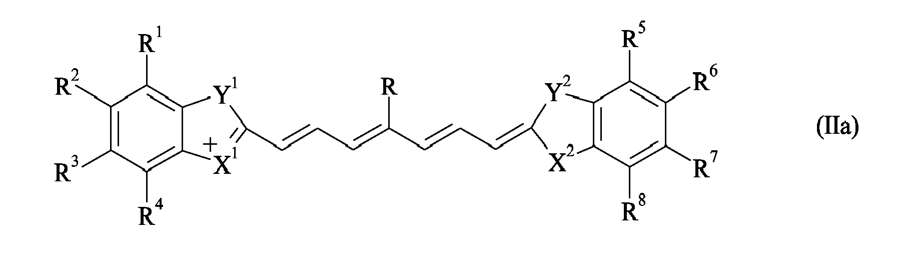 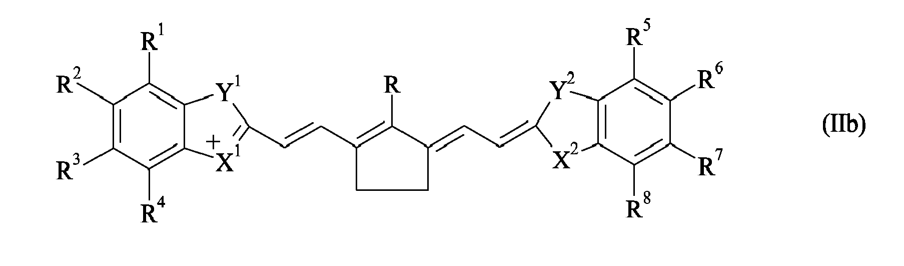 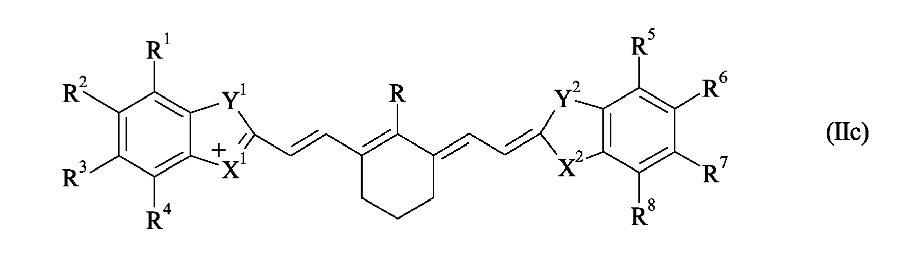 wherein
X¹, X², Y¹, Y², R, R¹ to R⁸ have the meaning as indicated above.

In a further preferred embodiment, X¹, X² are N(R¹³), Y¹ and Y² are C(CH₃)₂ and R¹³ is the same or different and has the meaning as indicated above.

Preferably, at least one R¹³ is present and is defined as being Ch-L-.

In case the compounds of the present invention are not sufficiently hydrophilic, it is possible to introduce a suitable hydrophilic group, preferably a sulfonic group.

Thus, another object of the present invention is a compound as described above which comprises at least one SO₃H or SO₃⁻ group.

In another preferred embodiment, one hydrogen atom in the chemical structure of the compounds described above is (formally) replaced by a group L'-T, wherein L' is a covalent bond or a linker and T is a biological detecting unit having a molecular weight of up to 30000 that bonds to specific cell populations or selectively or non-selectively to receptors, or accumulates in tissues or tumors or generally stays in the blood.

More preferred R or a hydrogen of R according to formulae II, IIa, IIb, IIc is replaced by the group L'-T.

The formal replacement of a hydrogen in the structure may be accomplished, e.g., by an esterification reaction of a carboxylic group (-COOH), resulting in (COOL'-T. Another example is the reaction of an amide group (C(O)NH₂), resulting in C(O)NH-L'-T. Further reactions are known in the art to come to the desired structures (see e.g. S. A. Hildebrandt et al., Bioconjugate Chem. 16 (2005), 1275-1281).

The linker L' may be the same or different to L. Examples for T are described in US-A 2001/055567. Preferred biological detecting units are biologically active peptides, proteins including antibodies, glycoproteins, lipids, sugars, PMA, DNA, RNA or parts thereof (so called probes). Preferred suitable targets are receptors, differentiation markers at the surface or in the cytoplasm of the cells, nucleonic acids (DNA, RNA) and compounds of the extracellular matrix.

The compounds of the present invention may further comprise a metal suitable for the at least one non-optical imaging technique coordinately bound to the chelating moiety Ch.

Preferably, the metal is Gd, Ga or In.

Another object of the present invention is a contrast agent for the at least bi-modal diagnostic imaging of a biological system, said at least bi-modal imaging comprising at least an optical and at least a non-optical imaging technique comprising a compound as defined above and a metal suitable for the at least one non-optical imaging technique coordinately bound to the chelating moiety Ch.

Suitable metals are described above.

These contrast agents can be used for the at least bi-modal diagnostic imaging of a biological system, said at least bi-modal imaging comprising at least an optical and at least a non-optical imaging technique.

The at least one optical imaging technique is preferably near-infrared fluorescence.

The at least one non-optical imaging technique is preferably selected from the group consisting of nucleomagnetic resonance imaging (NMRI), positron emission tomography (PET), single photon emission computer tomography (SPECT), and scintigraphy.

Another preferred embodiment of the present invention are the compounds defined above as such. Preferred compounds are defined by formula II as described above, wherein Ch-L is selected from the formulae IIIb and IIId as described above.

Yet another embodiment of the present invention is an in-vivo diagnostic method for the at least bi-modal diagnostic imaging of a biological system, said at least bi-modal imaging comprising at least an optical and at least a non-optical imaging technique using a contrast agent as described above.

At least dual contrast agents can be used to assess surrogate markers of vascularisation (e.g. increased angiogenesis in tumors) or alternatively as specific contrast agents that bind to a certain pathology after coupling to biologically active peptides, proteins or such. Pathologies can be neoplastic, degenerative and inflammatory processes.

In basic research on small animals dual contrast agents are favourably suited to learn more about quantification of optical signals deriving from deeper tissues in optical tomography.

For the clinical use optical imaging is limited by its tissue penetration. Using double contrast agents it is thus plausible that pathologies, which are localised deeper in the body of humans are first localised by MRI, SPECT, PET or scintigraphy. During subsequent biopsy or surgery, however, when the body has been opened optical near infrared imaging enables the accurate and fast localisation of the pathology within the tissue. In case of tumors this also includes the assessment of safety margins. Hereby, optical imaging will guide the surgent in removing the entire lesion, which is essential for a positive patient outcome.

Also in case of superficial lesions or those that can be reached by endoscopic procedures (e.g. colon adenoma, gastric cancer, lung cancer) nuclear medicine techniques or MRI may be preferable for screening and first localisation, while optical imaging will play the major role for biopsy and tissue removal.

The present invention is now described in more detail by way of examples which do not limit the scope of the present invention.

### Examples

### Example 1 - Synthesis:

General: The commercially available chemicals used for the synthetic work were of reagent grade quality and were used as obtained. 1-(3-Aminopropyl)-2,3,3-trimethyl-3H-indoleninium bromide hydrobromide **1** and 5-Carboxy-1-(4-sulfobutyl)-2,3,3-trimethyl-3H-indolenin **2** were synthesized as described in the literature [1, 2] and analyzed by TLC, HPLC, NMR and MS and regarded pure enough to be used without further purification.

**Purification and Spectroscopic Analysis:** Thin layer chromatography (TLC) was performed on Polygram SIL G/UV₂₅₄ plates (Macherey-Nagel). Flash chromatography was performed on silica gel 60 (70-230 mesh, Merck). Nuclear magnetic resonance (NMR) spectra were obtained with a Bruker 250 MHz spectrometer. Chemical shifts reported in ppm using TMS as a standard. High performance liquid chromatography (HPLC) was performed on an Agilent Series 110 instrument using a Chromolite Performance RP 18 E 100-4.6 mm (Merck), eluent A water 0.1 % TFA eluent B acetonitril 0.1 % TFA using a linear gradient 0 to 100% B in 5 min. Mass spectra (MS) were measured using a MS-ESI instrument. Absorption and emission spectra were determined using

### Synthesis of cyanine dyes containing DOTA:

**(1,4,7,10-Tetraazacyclodecan-1,4,7,10-tetraacetic acid)-4-nitrophenylester (DOTA-PNP):** *1,4,7,10-Tetraazacyclodecan-1,4,7,10-tetraacetic acid)-4-nitrophenylester (DOTA-PNP)* **3** *was synthesized in a slightly modified procedure to the one in the literature [3] facilitating the preparation of larger quantities of the compound which, however, contains minor impurities.* DOTA ^{·}0.31 H₂O (2.0 g, 4.88 mmol) was dissolved in water (40 ml). A solution of 4-nitrophenol (678 mg, 4.88 mmol) in acetonitrile (40 ml) was added followed by slow addition of dicyclohexylcarbodiimid (1.01g, 4.88 mmol) in pyridine (10 ml). The reaction mixture was stirred 90 min at room temperature and then evaporated. The residue was evaporated with toluene to remove traces of pyridine redissolved in CH₂Cl₂/MeOH 1:1, and purified by chromatography (silica gel; gradient of CH₂Cl₂/MeOH 1:1 to pure methanol to MeOH/water 8:2, all containing 0.05 % TFA). Fractions of similar purity (TLC, HPLC) were combined and evaporated to give 1.28 g of **3** of approximately 85 % purity which was used as such for the further experiments.

**1-(3-DOTA-Amidopropyl)-2,3,3-trimethyl-3H-indoleninium bromide:** To a solution of **1** (790 mg, 2.1 mmol) and N-ethyldiisopropylamine (1.5 ml) in acetonitrile (10 ml) was added a solution of DOTA-PNP **3** (1.28 g, 2.4 mmol) in N,N-dimethylformamide (10 ml). The solution stirred over night at room temperature. The solvents were evaporated in vacuum and the residue stirred with diethylether, the solvent decanted and the semisolid residue dissolved in methanol containing 5 % water. Silica was added to the solution and the slurry applied to a column of silica eluting dichloromethane containing 40 to 100 % methanol then with methanol containing 0 to 50 % water and 0.05 % TFA. Fractions were analyzed by TLC and HPLC and combined to give 1.0 g of compound 4 of approx. 75 % purity (HPLC), which was used without further purification.

**{[3-(anilinomethylene)-2-chloro-1-cyclohexen-1-yl] ethenyl}-5-carboxy-3,3-dimethyl-1-(4-sulfobutyl)-3*H*-indolium** (5): To a solution of **2** (678 mg, 2 mmol) and sodium acetate (550 mg, 6.5 mmol) in methanol (125 ml) was added N-((3-aminomethylene)-2-chloro-1-cyclohexen-1-yl)methylene)aniline monohydrochlorid (1.077 g, 3 mmol) and the reaction heated at reflux for 2 hs. TLC (30 % methanol in dichlormethane) shows the formation of a turkis product. The solvent was evaporated and the residue purified on silica eluting with dichloromethane containing 0 to 40% of methanol to give compound **5** (850 mg) as a dark solid.

**2-(2-(3-(2-(5-carboxy-3,3-dimethyl-1-(4-sulfobutyl)-1,3-dihydro-2*H* indol-2-ylidene)ethylidene)-2-chlorocyclohex-1-en-1-yl)vinyl)-3,3-dimethyl-1-(3-(((4,7,10-tris(carboxymethyl)-1,4,7,10-tetraazacyclododecan-1-yl)acetyl)amino)propyl)-3*H-***indolium (6): To **4** (100 mg, 0.15 mmol), **5** (170 mg, 0.3 mmol) and sodium acetate (400 mg, 4.8 mmol) in a mixture of ethanol (100 ml) and methanol (10 ml) was added acetic acid (2 ml) and the resulting solution was heated at 90-100 °C for 2 hs. TLC (CH₂Cl₂/MeOH 7:3) shows the formation of a green product. The solvents were evaporated and the product purified by chromatography (silica gel; gradient of CH₂Cl₂/MeOH 8:2 to pure methanol) yielding in pure **6** (217 mg). *For preparation of the metal complexes and for radioactive labelling the dye was further purified by preparative HPLC.*

**Gd(III)-complex of compound 6:** Crude compound **6** (35 mg) was dissolved in a mixture of DMSO (400 µl), MeOH (400 µl), 0.4 M sodium acetate buffer pH 5 (2 ml) and water (1 ml). A solution of GdCl₃ ^{·}6H2O (56 mg) in sodium acetate buffer (400 µl) was added and the reaction mixture heated at 60° in an oil bath for 35 min. HPLC showed complete conversion of compound **6**. The complex was purified by preparative RP-HPLC (Chromolite Column, Merck, Germany), pure fractions combined and lyophilized to give pure compound 6 (13.3 mg) as a green solid.

**¹¹¹In(III)-complex of compound 6:** In order to radiolabel compound **6** with approximately 75 MBq/mg HPLC purified compound **6** (8 mg) was dissolved in DMSO (1.2 ml). From this solution an aliquot (300 µl) was labelled. The aliquot of 300 µl was diluted with 300 µl of 0.4 M sodium acetate buffer pH 5 and 660 MBq ¹¹¹InCl₃ in diluted HCl (100 µl). 0.4 M sodium acetate buffer pH 5 (200 µl) was added and the solution heated at 60° for 10 min. Analytical HPLC proved complete complexation of ¹¹¹In(III) with compound **6**. Then the remaining aliquot (900 µl) of uncomplexed compound 6 was added to the labelled aliquot and 0.5 M phosphate buffer pH 8 (500 µl) and water (500 µl) were added to give compound **6** (8 mg) dissolved in 3.8 ml labelled with 608 MBq ¹¹¹In(III) containing no free ¹¹¹In(III) (as confirmed by HPLC).
[1] Narayanan, N. and Patonay, G. (1995) A new method for synthesis of heptamethine cyanine dyes: Synthesis of new near-infrared fluorescent labels. J. Org. Chem. 60, 2391-2395.
[2] Terpetschnig, E., Szmacinski, H., Ozinskas, A. and Lakowicz, J. R. (1994) Synthesis of Squaranine-N-hydroxysuccinimid esters and their biological application as long-wavelength fluorescent labels. Analytical Biochemistry 217, 197-204.
[3] Mier, W., Hoffend, J., Krämer, S., Schuhmacher, J., Hull, W. E., Eisenhut, M., and Haberkorn, U. (2005) Conjugation of DOTA using isolated phenolic active esters: the labeling and biodistribution of albumin as blood pool marker. Bioconjugate Chem. 16, 237-240.

### Example 2 - Examples for the use in non-invasive imaging:

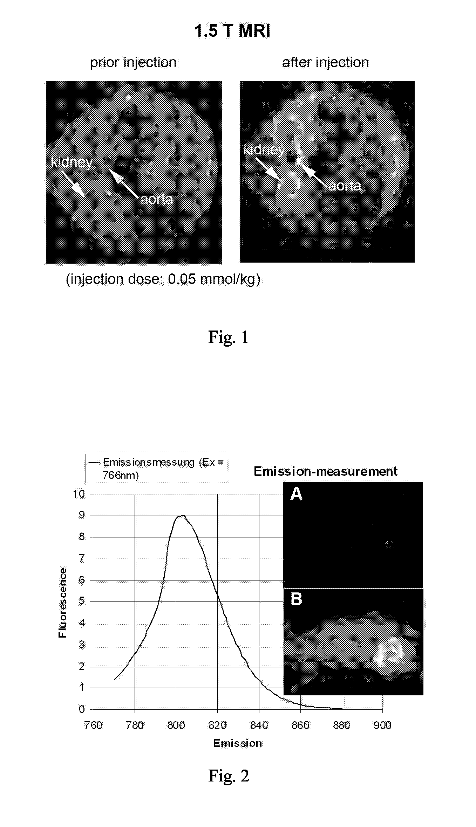

Figure 1 shows the transversal T1w-images of a nude mouse at the height of the kidneys prior and after injection of the probe loaded with gadolinium (dose: 0.05 mmol Gd/kg) (see example 1). The enhancement of signal intensity in aorta and kidneys is clearly visible.

Figure 2 shows the emission of the probe (Ext: 766 nm) is clearly in the near infrared range at approximately 800 nm. Images of a tumor bearing nude mouse before (A) and after (B) injection of the probe (concentration: 0.1 µmol/kg, see example 1) clearly show an enhancement in the tumor and the mouse.

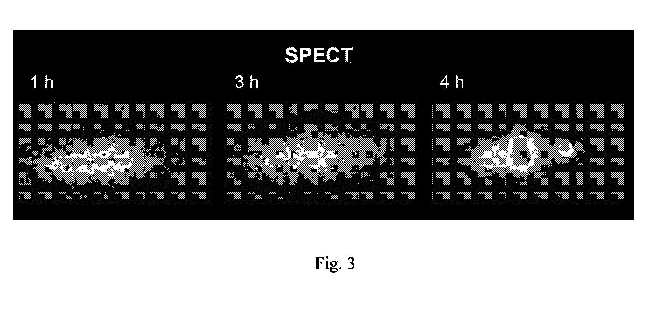

Figure 3 shows planar images of a nude mouse after injection of the probe loaded with ¹¹¹In (see example 1) captured with a combined SPECT-Optical Tomograph. After 1 hour high activity is still observed along the large arteries and veins. After 3 hours the probe is distributed unspecifically and after 4 hours it is accumulated in the kidney and the urine bladder indicating renal excretion.

## Claims

1. Use of a compound of formula I
Ch-L-D (I),
wherein D is a cyanine dye, L is a covalent bond or a linker and Ch is a metal chelating moiety or a physiologically acceptable salt thereof, for the preparation of a contrast agent for the at least bi-modal diagnostic imaging of a biological system, said at least bi-modal imaging comprising at least an optical and at least a non-optical imaging technique.

2. The use according to claim 1, wherein the compound is represented by formula II 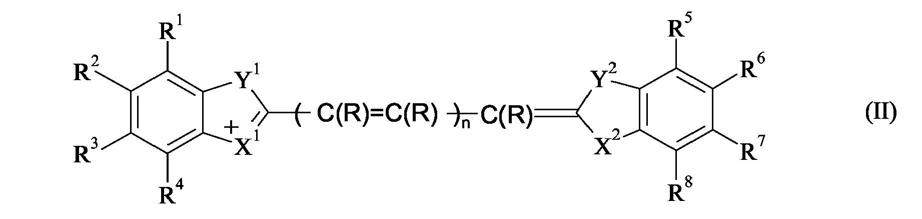 wherein
X¹ is selected from the group consisting of O, N(R¹³), C(R¹⁴)-C(R¹⁵)=O, and C(R¹⁴)-C(R¹⁵)=N(R¹³);
X² is selected from the group consisting of O, and N(R¹³);
Y¹, Y² are independently selected from the group consisting of a covalent single bond, -C(R⁹R¹⁰)-, O, S, -C(R⁹R¹⁰)-C(R¹¹R¹²)-, and -C(R⁹)=C(R¹¹)-;
n is 0, 1, 2, 3, 4 or 5, preferably 1, 2, or 3;
each R is independently selected from the group consisting of hydrogen, halogen, OH, NH₂, C(O)OH, C(O)NH₂, SO₃H, NO₂, CN, Ch-L-, and C₁₋₆ alkyl, wherein C₁₋₆ alkyl is optionally substituted with one or more halogen, OH, NH₂, C(O)OH, C(O)NH₂, SO₃H, NO₂, CN;
optionally two R groups in 1,3-cis position jointly form together with the carbons to which they are attached to a cycle selected from the group consisting of cyclopentene, cyclopentadiene, cyclohexene, and cyclohexadiene, wherein the cycle is optionally substituted with one or more substituents, which are the same or different, selected from the group consisting of halogen, OH, NH₂, C(O)OH, C(O)NH₂, SO₃H, NO₂, CN, Ch-L-, and C₁₋₆ alkyl, wherein C₁₋₆ alkyl is optionally substituted with one or more halogen, OH, NH₂, C(O)OH, C(O)NH₂, SO₃H, NO₂, CN;
R¹, R², R³, R⁴, R⁵, R⁶, R⁷, R⁸, R⁹, R¹⁰, R¹¹, R¹², R¹⁴, R¹⁵ are independently selected from the group consisting of hydrogen, halogen, OH, NH₂, C(O)OH, C(O)NH₂, SO₃H, N0₂, CN, Ch-L-, and C₁₋₆ alkyl, wherein C₁₋₆ alkyl is optionally substituted with one or more halogen, OH, NH₂, C(O)OH, C(O)NH₂, SO₃H, NO₂, CN;
optionally one or more pairs selected from the group consisting of R¹/R², R²/R³, R³/R⁴, R⁵/R⁶, R⁶/R⁷, and R⁷/R⁸ jointly form together with the carbon atoms to which they are attached to a cycle selected from the group consisting of phenyl, naphthyl, heterocycle and heterobicycle;
each R¹³ is independently selected from the group consisting of hydrogen, C(O)OH, C(O)NH₂, Ch-L-, and C₁₋₆ alkyl, wherein C₁₋₆ alkyl is optionally substituted with one or more halogen, OH, NH₂, C(O)OH, C(O)NH₂, SO₃H, N0₂, CN;
provided that one of the groups R, R¹ to R¹⁵ is -L-Ch, wherein L and Ch have the meaning as indicated in claim 1.

3. The use according to claim 1 or 2, wherein L is a covalent bond or C₁₋₆ alkyl, which is optionally interrupted by one or more atoms or functional groups selected from the group consisting of -S-, -O-, -NH-, -C(O)O-, and -C(O)NH-, and optionally substituted with one or more substituents independently selected from the group consisting of halogen, OH, NH₂, C(O)OH, C(O)NH₂, SO₃H, NO₂, and CN.

4. The use according to any of claims 1 to 3, wherein Ch-L is selected from the group consisting of 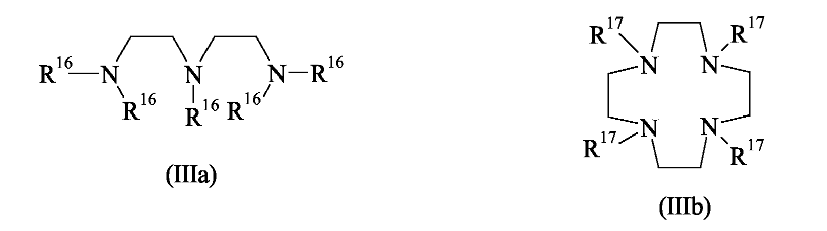 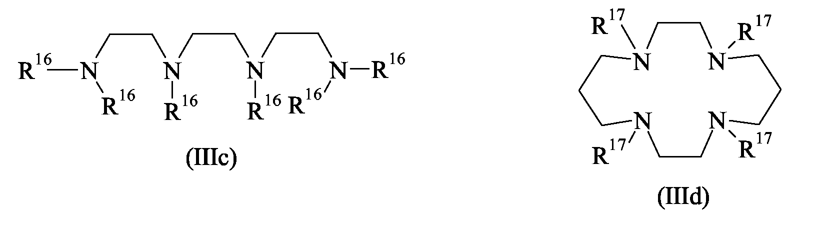 wherein
each R¹⁶ is independently selected from the group consisting of hydrogen, CH₂C(O)OH, CH₂C(O)NH₂, CH₂C(O)NHCH₃, CH₂C(O)NHCH₂Ph, CH₂PO₃H, CH₂P(CH₂Ph)O₂H, CH₂SO₃H, CH₂CH₂OH, C(O)R¹⁸, C(O)CH₂NHC(O)R¹⁸, and deprotonated derivatives thereof;
each R¹⁷ is independently selected from the group consisting of hydrogen, CH₂C(O)OH, CH(CH₂OCH₂Ph)C(O)OH, CH₂C(O)NH₂, CH₂C(O)NHCH₃, CH₂C(O)NHCH₂Ph, CH₂PO₃H, CH₂P(CH₂Ph)O₂H, CH₂SO₃H, CH₂CH₂OH, CH₂CH(OH)CH₃ and deprotonated forms thereof;
R¹⁸ is selected from the group consisting of 3-hydroxy-pyridin-2-on-4-yl, 5-hydroxy-pyrimidin-4-on-6-yl, 1-oxo-pyridin-2-on-6-yl, 1-hydroxy-phen-2-yl, 1,2-dihydroxy-phen-3-yl, wherein R¹⁸ is optionally substituted with one or more further substituents independently selected from the group consisting of CH₃, CH₂CH₂OH, CH₂CH₂OCH₃, C(O)NH₂, and C(O)NHCH₃,
provided that one hydrogen of the compound according to one of the formulae IIIa to IIId is replaced by L and L has the meaning as indicated in claim 1.

5. The use according to any of claims 2 to 4, wherein the compound is represented by formula IIa, IIb or IIc 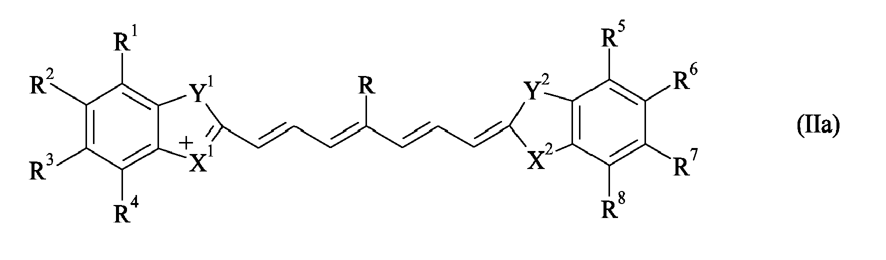 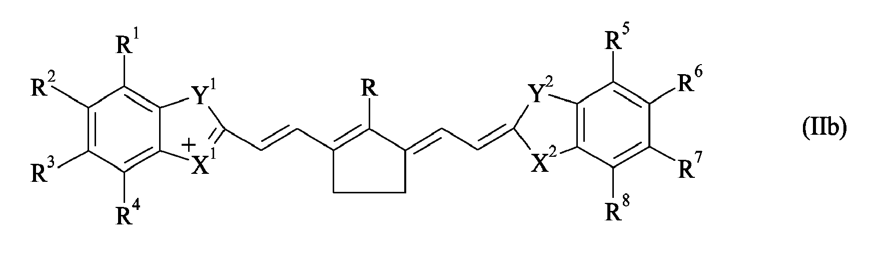 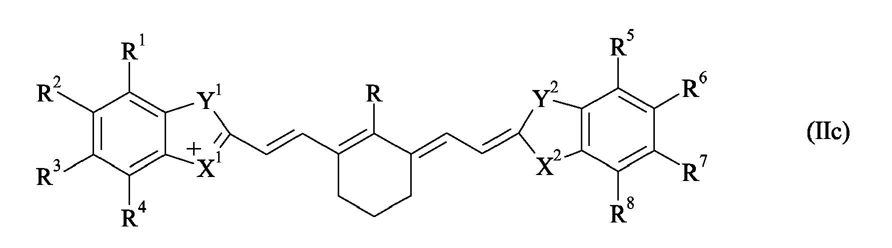 wherein X¹, X², Y¹, Y², R, R¹ to R⁸ have the meaning as indicated in claim 2.

6. The use according to any of claims 2 to 5, wherein X¹, X² are N(R¹³), Y¹ and Y² are C(CH₃)₂ and R¹³ is the same or different and has the meaning as indicated in claim 2.

7. The use according to any of claims 2 to 6, wherein at least one R¹³ is present and is defined as being Ch-L-.

8. The use according to claim 4, wherein Ch-L is selected from the formulae IIIb and IIId.

9. The use according to any of claims 1 to 8, wherein the compound comprises at least one SO₃H or SO₃⁻ group.

10. The use according to any of claims 1 to 9, wherein one hydrogen atom in the chemical structure of the compound is replaced by a group L'-T, wherein L' is a covalent bond or a linker and T is a biological detecting unit having a molecular weight of up to 30000 that bonds to specific cell populations or selectively or non-selectively to receptors, or accumulates in tissues or tumours or generally stays in the blood.

11. The use according to any of claims 1 to 10, wherein the compound further comprises a metal suitable for the at least one non-optical imaging technique coordinately bound to the chelating moiety Ch.

12. The use according to claim 11, wherein the metal is Gd, Ga or In.

13. The use according to any of claims 1 to 12, wherein the at least one optical imaging technique is near infrared fluorescence.

14. The use according to any of claims 1 to 13, wherein the at least one non-optical imaging technique is selected from the group consisting of nuclear magnetic resonance imaging (NMRI), positron emission tomography (PET), single photon emission computer tomography (SPECT), and scintigraphy.

15. A contrast agent for the at least bi-modal diagnostic imaging of a biological system, said at least bi-modal imaging comprising at least an optical and at least a non-optical imaging technique comprising a compound as defined in one of the claims 1 to 10 and a metal suitable for the at least one non-optical imaging technique coordinately bound to the chelating moiety Ch.

16. The contrast agent according to claim 15, wherein the metal is Gd, Ga or In.

17. Use of a contrast agent according to claim 15 or 16 for the at least bi-modal diagnostic imaging of a biological system, said at least bi-modal imaging comprising at least an optical and at least a non-optical imaging technique.

18. The use according to claim 17, wherein the at least one optical imaging technique is near infrared fluorescence.

19. The use according to claim 17 or 18, wherein the at least one non-optical imaging technique is selected from the group consisting of nuclear magnetic resonance imaging (NMRI), positron emission tomography (PET), single photon emission computer tomography (SPECT), and scintigraphy.

20. A compound as defined by formula II of claim 2, wherein Ch-L is defined as indicated in claim 8.

21. An in-vivo diagnostic method for the at least bi-modal diagnostic imaging of a biological system, said at least bi-modal imaging comprising at least an optical and at least a non-optical imaging technique using a contrast agent according to claim 15 or 16.
